# EUROPEAN PATENT SPECIFICATION

(11) **EP 4 085 254 B1**
(45) Date of publication and mention of the grant of the patent: **05.06.2024**
(21) Application number: 20842240.2
(22) Date of filing: 29.12.2020
(51) Int. Cl.: G01N 33/68

(54) **ADA-RESPONSE SPECIFICATION ASSAY**
ADA-REAKTIONSSPEZIFIKATIONSASSAY
ESSAI DE SPÉCIFICATION DE LA RÉPONSE ADA

(30) Priority: 02.01.2020 EP 20150136
(43) Date of publication of application: 09.11.2022
(73) Proprietor: F. Hoffmann-La Roche AG, 4070 Basel (CH)
(72) Inventor: BECKMANN, Roland, 82377 Penzberg (DE); MOESSNER, Ekkehard, 8952 Schlieren (CH); STUBENRAUCH, Kay-Gunnar, 82377 Penzberg (DE)
(74) Representative: Skolaut, Alexander
(86) International application number: PCT/EP2020/087968
(87) International publication number: WO 2021/136773

(56) References cited:
- WO-A1-2018/178307
- STUBENRAUCH KAY ET AL: "Epitope characterization of the ADA response directed against a targeted immunocytokine", JOURNAL OF PHARMACEUTICAL AND BIOCHEMICAL ANALYSIS, ELSEVIER B.V, AMSTERDAM, NL, vol. 114, 5 June 2015 (2015-06-05), pages 296-304, XP029260164, ISSN: 0731-7085, DOI: 10.1016/J.JPBA.2015.05.029 cited in the application
- STUBENRAUCH K ET AL: "Evaluation of a biosensor immunoassay for simultaneous characterization of isotype and binding region of human anti-tocilizumab antibodies with control by surrogate standards", ANALYTICAL BIOCHEMISTRY, ACADEMIC PRESS, AMSTERDAM, NL, vol. 390, no. 2, 15 July 2009 (2009-07-15) , pages 189-196, XP026145499, ISSN: 0003-2697, DOI: 10.1016/J.AB.2009.04.021 [retrieved on 2009-04-18]
- Zhongyu Zhu ET AL: "Construction of a Large Naive Human Phage-Displayed Fab Library Through One-Step Cloning" In: "Antibody-drug conjugates; IN: Methods in Molecular Biology; ISSN 1064-3745; Vol. 1045", 1 January 2009 (2009-01-01), Humana Press, US, XP055388109, ISBN: 978-1-62703-541-5 vol. 525, pages 129-142, DOI: 10.1007/978-1-59745-554-1_6, the whole document
- BRODERS OLAF ET AL: "Novel bioanalytical method for the characterization of the immune response directed against a bispecific F(ab) fragment", BIOANALYSIS, vol. 12, no. 8, 1 April 2020 (2020-04-01), pages 509-517, XP55772307, London, UK ISSN: 1757-6180, DOI: 10.4155/bio-2020-0064
- JOHN H SLOAN ET AL: "An innovative and highly drug-tolerant approach for detecting neutralizing antibodies directed to therapeutic antibodies", BIOANALYSIS, vol. 8, no. 20, 1 October 2016 (2016-10-01), pages 2157-2168, XP055712409, London, UK ISSN: 1757-6180, DOI: 10.4155/bio-2016-0161

## Description

The current invention is in the field of assays for the determination and quantification of an anti-drug response to a therapeutic antibody (drug). With the assay as reported herein, it is possible to determine the spatial binding region of the anti-drug antibodies (ADA) in a sample, especially a plasma sample.

### Background

Many biotherapeutics in development today are designed to enable several functionalities and complex mechanisms of action. This can be achieved by incorporation of multiple functional domains and/or engineering of specific amino acid sequences.

Nevertheless, all therapeutic proteins have the potential to induce anti-drug antibodies (ADA) in the recipient animal (preclinical) or human (clinical) organism. Clinically relevant ADA can affect efficacy and/or safety of a biological therapeutic. Immunogenicity assessment strategy evaluates binding and neutralizing ADA and the need for additional characterization (e.g., epitope, titer and so on) is determined using a risk-based approach.

ADA characterization can affect the interpretation of the risk profile of a given therapeutic, and offers insight into opportunities for risk mitigation and management.

This is of special relevance for the treatment of chronic diseases, such as, for example, AMD and DME, as the treatment is intended to be applied for months or even years. If an immune response occurs, it is important to understand, against which region or domain of the molecule the response is directed. This way, information is gathered, whether e.g. an engineered part of the molecule, which could be re-engineered (to e.g. remove potential T-cell epitopes), is immunogenic, or if the immunogenicity response is directed against the antigen-binding parts (HVRs) of the biotherapeutic, an essential intrinsic property of the molecule. An appropriate risk mitigation strategy can then be developed.

Established methods for immunogenicity response or anti-drug antibody (ADA) characterization are classical domain competition and domain detection assays (DCA, DDA; see, e.g., Hoofring, S.A., et al., Bioanal. 5 (2013) 1041-1055; Gorovits, B., et al., J. Immunol. Meth. 408 (2014) 1-12; Stubenrauch, K., et al., J. Pharm. Biomed. Anal. 114 (2015) 296-304). Here, the drug candidate is cleaved into single domains or specific sub fragments are recombinantly expressed, to allow the development of domain specific ADA assays. These widely used approaches are of particular value for large protein molecules, such as IgG or IgG-like modalities and fusion proteins. For smaller biotherapeutics, such as single-chain Fv fragments (scFv), Fab fragments or bispecific Fabs, the above approaches have limitations, as these molecules often cannot be easily cleaved into subdomains or these subdomains have stability liabilities. Therefore, alternative, more generally applicable approaches are needed.

The drawbacks of competitive/inhibition-based ADA detection methods, especially in view of mixed-affinity and mixed-paratope ADA responses, has been reported by Hoofring, et al. (Bioanal. 5 (2013) 1041-1055) and Stubenrauch, et al. (J. Pharm. Biomed. Anal. 10 (2015) 296-304.

WO 2018/178307 reported improved immunogenicity assays that are capable of distinguishing neutralizing anti-drug antibodies from non-neutralizing anti-drug antibodies. In more detail, it is reported an assay/method that is performed in the presence of a sufficient amount of, and preferably an excess of a null variant of the immunoglobulin single variable domain (ISV) based drug, such that most and preferably all of the non-neutralizing ADAs, in particular, ADAs that bind to the framework sequences of the ISV(s) that are present in the ISV-based drug, bind to the null variant in the assay reaction mix rather than to the ISV-based drugs, so that essentially only neutralizing ADAs against the ISV-based drug are detected or measured (even when the sample also contains non-neutralizing ADAs against the ISV-based drug). In the null variant only individual residues have been changed but no complete CDR sequence.

Stubenrauch, et al., reported about the epitope characterization of the ADA response directed against a targeted immunocytokine (J. Pharmaceut. Biochem. Anal. 114 (2015) 296-304.

Stubenrauch, et al. reported about the evaluation of a biosensor immunoassay for simultaneous characterization of isotype and binding region of human anti-Tocilizumab antibodies with control by surrogate standards (Anal. Biochem. 390 (2009) 189-196.

Zhu, et al., reported about construction of a large naive human phage-displayed Fab library through one-step cloning (Methods. Mol. Biol. 1045 (2009) 129-142).

Broders, O., et al., reported a novel bioanalytical method for the characterization of the immune response directed against a bispecific F(ab) fragment (Bioanal. 12 (2020) 509-517).

### Summary of the Invention

Many biologics, offering tailor-made properties and functionalities for a wide range of indications, are currently in development. During preclinical development of therapeutic antibodies, it is especially important to assess the immunological response against the compound amongst others with respect to its potential impact on the pharmacokinetic profile, target binding properties, safety and efficacy.

The current invention is directed to an analytical method that allows to classify the immunogenicity of different domains of a therapeutic antibody, such as, e.g., a bispecific Fab, in an experimental animal, such as, e.g., a cynomolgus monkeys. In the method according to the current invention, a molecular engineering approach is combined with a domain detection assay. The invention is based, at least in part, on the finding that by replacement of the antigen-binding regions, i.e. the paratope, in a Fab with human germline sequences it was possible to discriminate between anti-drug antibodies (ADAs) directed against either one of the two target-binding paratopes as well as the constant part of the bispecific Fab. The method according to the invention has advantageous properties, especially for smaller biotherapeutics, such as Fab or scFv fragments. The method allows reliable characterization of the ADA response and a better understanding of preclinical immunogenicity using easy to generate tool variant antibodies.

Without being bound by this theory, the method according to the current invention provides improvements in view of prior know methods, especially a simpler feasibility as well as a higher reliability with regard to false negative results. The second advantage is based, at least in part, in the direct capture of a variant of the therapeutic antibody in which at least one complete HVR has been replaced with a complete, non-binding HVR, whereby the binding of the respective anti-HVR anti-drug antibodies, i.e. in the wrong place, is prevented. Methods using variants, wherein only a part of or only individual residues in the HVR are replaced to generate non-target binding therapeutic antibodies and, thus, wherein a part of the paratope is maintained although target binding is abolished, are more prone to false negative results. This is because neutralizing antibodies binding specifically to remaining residues of the paratope of the therapeutic antibody can still interfere.

The current invention is directed to a bioanalytical method for immunogenicity response characterization after application of a therapeutic antibody to an experimental animal, such as a cynomolgus monkey.

The invention is based, at least in part, on the finding that by the combination of a molecular engineering approach with an efficient and easy to setup domain detection assay it is possible to determine and quantify the spatial location of the anti-drug response on the therapeutic antibody in an easy and reliable way. It has been found that by replacement of the antigen-binding regions by human germline sequences the assay was able to discriminate between anti-drug antibodies (ADAs) directed against the target-binding paratopes, as well as the constant part of the therapeutic antibody. It has further been found that in combination with a timely and thorough reagent development, the assay according to the invention is advantageous especially for smaller therapeutic antibodies, such as scFvs and Fabs. With the assay according to the current invention, a reliable characterization of the ADA response and a better understanding of preclinical immunogenicity can be achieved. The method according to the current invention can be applied to any therapeutic antibody or antibody format to obtain ADA epitope information as long as the paratope is formed by a VH/VL-pair, whereby the efforts to generate the required reference antibodies with removed paratope is reduced compared to methods known in the art.

In more detail, herein is reported a novel bioanalytical method for characterization of the immunogenicity response against a therapeutic antibody, especially a bispecific antibody Fab fragment, by back mutation of the respective antigen-binding sites/paratope to human germline sequences, thereby abolishing the binding specificity, i.e., for example, for either antigen-1, antigen-2 or both antigens. The domain detection assay according to the current invention is based on these variants of the therapeutic antibody. By using these variants, it is possible to discriminate between anti-drug antibodies (ADAs) directed against the first and the second specificity, both specificities, and the constant part of the molecule. The improvement lies, amongst other things, in the reduction of the efforts required to generate the paratope variants of the therapeutic antibody.

One aspect of the invention is a method for determining the epitope of an anti-drug antibody, which is specifically binding to a therapeutic antibody specifically binding to a therapeutic target, comprising the following steps:
a) separately incubating aliquots of a sample, which comprises serum and the antibody specifically binding to a therapeutic antibody, separately with
   i) at least a Fab fragment of the therapeutic antibody, and
   ii) at least a Fab fragments of the therapeutic antibody, in which all hypervariable regions (HVRs) have been replaced with germline or non-binding HVRs,
   and detecting the binding or non-binding of the antibody specifically binding to a therapeutic antibody to the at least a Fab fragment in any of i) to ii),
   and
b) determining the epitope of the antibody specifically binding to a therapeutic antibody to be
   - in the HVRs that have been replaced in ii) if binding is detected in i)
      and
      non-binding is detected in ii),
   - in the HVR-framework junction of the HVRs that have been replaced in ii) or in the framework-regions or in the constant domains if
      binding is detected in i) and ii).

In one embodiment of the method according to the invention, the non-binding HVR or the germline HVR is obtained from the same germline as the Framework-regions of the therapeutic antibody are.

In one embodiment of the method according to the invention, the therapeutic antibody is a mono- or bispecific Fab.

In one embodiment of the method according to the invention
- the therapeutic antibody is a bispecific Fab, wherein the first paratope is formed by the HVRs H-1, H-3 and L-2 and the second paratope is formed by the HVRs H-2, L-1 and L-3,
- in step a) step ii) is
   the therapeutic antibody in which HVRs H-1, H-3 and L-2 have been replaced with non-binding HVRs,
- step a) further comprises
   vi) the therapeutic antibody in which HVRs H-2, L-1 and L-3 have been replaced with non-binding HVRs,
- step b) is
   determining the epitope of the antibody specifically binding to a therapeutic antibody to be
   - in the first paratope of the bispecific Fab if binding is detected in i) and vi),
      and
      non-binding is detected in ii),
   - in the second paratope of the bispecific Fab if binding is detected in i) and ii),
      and
      non-binding is detected in vi),
- in the HVR-Framework region-junction of the first paratope if binding is detected in i) and vi)
   and
   non-binding is detected in ii),
- in the HVR-Framework region-junction of the second paratope if binding is detected in i) and ii)
   and
   non-binding is detected in vi), .

In all aspects and embodiments of the current invention, no soluble variant of the therapeutic antibody or fragment thereof is added to the sample or the aliquot of the sample, prior to step a), i.e. prior to the incubation with the immobilized at least Fab of the therapeutic antibody or a paratope-modified variant thereof.

In all aspects and embodiments, the therapeutic antibody comprises a VH/VL-pair that forms the therapeutic target-binding paratope.

In all aspects and embodiments, the paratope of the therapeutic antibody binding to the therapeutic target is no single domain antibody.

### Detailed Descrintion of Embodiments of the Invention

### DEFINITIONS

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. The methods and techniques of the present disclosure are generally performed according to conventional methods well known in the art. Generally, nomenclatures used in connection with, and techniques of biochemistry, enzymology, molecular, and cellular biology, microbiology, genetics and protein and nucleic acid chemistry and hybridization described herein are those well-known and commonly used in the art.

Unless otherwise defined herein the term "comprising of" shall include the term "consisting of'.

The term "about" as used herein in connection with a specific value (e.g. temperature, concentration, time and others) shall refer to a variation of +/- 1 % of the specific value that the term "about" refers to.

The term "antibody" is used herein in the broadest sense and encompasses various antibody structures, including but not limited to monoclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')2; diabodies; linear antibodies; single-chain antibody molecules (e.g., scFv, and scFab); single domain antibodies (dAbs); and multispecific antibodies formed from antibody fragments.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. In certain aspects, the antibody is of the IgG₁ isotype. In certain aspects, the antibody is of the IgG₁ isotype with the P329G, L234A and L235A mutation to reduce Fc-region effector function. In other aspects, the antibody is of the IgG₂ isotype. In certain aspects, the antibody is of the IgG₄ isotype with the S228P mutation in the hinge region to improve stability of IgG₄ antibody. The heavy chain constant domains that correspond to the different classes of immunoglobulins are called α, δ, ε, γ, and µ, respectively. The light chain of an antibody may be assigned to one of two types, called kappa (κ) and lambda (λ), based on the amino acid sequence of its constant domain.

"Framework" or "FR" refers to variable domain residues other than complementary determining regions (CDRs). The FR of a variable domain generally consists of four FR domains: FR1, FR2, FR3, and FR4. Accordingly, the CDR and FR sequences generally appear in the following sequence in VH (or VL): FR1-CDR-H1(CDR-L1)-FR2-CDR-H2(CDR-L2)-FR3-CDR-H3(CDR-L3)-FR4.

The terms "full length antibody", "intact antibody", and "whole antibody" are used herein interchangeably to refer to an antibody having a structure substantially similar to a native antibody structure or having heavy chains that contain an Fc region as defined herein.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three complementary determining regions (CDRs). A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively.

The term "hypervariable region" or "HVR" as used herein refers to each of the regions of an antibody variable domain which are hypervariable in sequence and which determine antigen binding specificity, for example "complementarity determining regions" ("CDRs"). These regions form the paratope or binding site.

Generally, antibodies comprise six antigen binding specificity determining regions: three in the VH (H1, H2, H3), and three in the VL (L1, L2, L3). Exemplary antigen binding specificity determining regions herein include:
(a) hypervariable loops (HVRs) occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));
(b) complementary determining regions (CDRs) occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991));
(a+b) HVRs combined with CDRs occurring at amino acid residues 24-34 (L1), 50-56 (L2), 89-97 (L3), 26-35 (H1), 50-65 (H2), and 95-102 (H3) (Chothia and Lesk, J. Mol. Biol. 196:901-917 (1987) + Kabat et al., Sequences of Proteins of Immunological Interest, 5th Ed. Public Health Service, National Institutes of Health, Bethesda, MD (1991));
   and
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)).

Unless otherwise indicated, the HVRs are determined according to Kabat et al., supra. One of skill in the art will understand that the antigen binding specificity determining regions designations can also be determined according to Chothia, supra, McCallum, supra, or any other scientifically accepted nomenclature system.

A "paratope" or "antigen binding site", as used interchangeably herein, refers to a part of an antibody, which recognizes and binds to an antigen. A paratope is formed by several individual amino acid residues from the antibody's heavy and light chain variable domains that are arranged in spatial proximity in the tertiary structure of the Fv region. The paratope of an endogenous human antibody is formed by the 6 CDR (complementarity determining region) loops, which recognize a complementary epitope on its antigen's surface. In case of antibodies specific for a small antigen (such as small molecules), only a few amino acids from some CDRs, but not all are involved in the antigen recognition. CDR amino acid residues involved in direct antigen contacts are called specificity determining regions (SDRs). By analysis of 3-dimensional structure of the antigen-antibody complex amino acid residues, directly contacting the antigen can be identified based on their distance. FR (framework) residues spaced between the CDRs can also participate in antigen recognition, but to a lesser extent (the surface of such regions may account for up to 15% of the antigen and antibody contact surface) (see, e.g., Altshuler, E.P., Chemie 50 (2010) 203-258; Bujotzek, A., et al., mAbs 8 (2016) 288-305).

In one embodiment, the therapeutic antibody in the method according to the invention is a bispecific Fab. In one embodiment, the bispecific Fab comprise two "non-overlapping" paratopes in one cognate VH/VL pair. By "non-overlapping" is meant that none of the amino acids that are comprised in one of the two paratopes is comprised in the other paratope.

A "bispecific Fab" is a bispecific antibody as disclosed in WO 2012/163520. In a bispecific Fab, a single pair of a VH domain and a VL domain specifically binds to two different epitopes, wherein one paratope comprises amino acid residues from CDR-H2, CDR-L1 and CDR-L3 and the other paratope comprises amino residues from CDR-H1, CDR-H3 and CDR-L2. Bispecific Fabs comprise two non-overlapping paratopes within a cognate VH/VL pair and may simultaneously bind to the two different epitopes. Bispecific Fabs and methods for their generation by screening of libraries comprising monospecific Fab fragments are disclosed in WO 2012/163520.

In one embodiment, the therapeutic antibody in the method according to the invention is a bispecific Fab. In one embodiment, the bispecific Fab specifically binds to a first antigen (antigen-1) and to a second antigen (antigen-2). In one embodiment, the bispecific Fab specifically binds to a first antigen (antigen-1) via its first paratope and to a second antigen (antigen-2) via its second paratope. In one embodiment, the bispecific Fab comprises a first paratope and a second paratope within one cognate pair of a variable light chain domain (VL domain) and a variable heavy chain domain (VH domain), wherein the first paratope comprises amino acid residues from CDR-H2, CDR-L1 and CDR-L3 of the antibody, wherein second paratope comprises amino acid residues from the CDR-H1, CDR-H3 and CDR-L2 of the antibody.

In one embodiment, the bispecific Fab binds to a first antigen and a second antigen and comprises a first paratope and a second paratope within one cognate pair of a VL domain and a VH domain, wherein the pair of the variable light chain domain and the variable heavy chain domain can simultaneously bind to the first antigen and the second antigen.

In one embodiment, the bispecific Fab binds to a first antigen and a second antigen and comprises a first paratope and a second paratope within one cognate pair of a VL domain and a VH domain, wherein none of the amino acids that are comprised in the first paratope are comprised in the second paratope.

The term "sample" as used herein denotes any biological matrix from which the determination of an ADA response can be made. Exemplary, but not limiting, samples are serum, plasma, aqueous humor, vitreous humor, retina tissue lysate, and tumor tissues. In one preferred embodiment, the sample is blood plasma.

The term "epitope" denotes the site on an antigen, either proteinaceous or non-proteinaceous, to which an antibody binds. Epitopes can be formed both from contiguous amino acid stretches (linear epitope) or comprise non-contiguous amino acids (conformational epitope), e.g. coming in spatial proximity due to the folding of the antigen, i.e. by the tertiary folding of a proteinaceous antigen. Linear epitopes are typically still bound by an antibody after exposure of the proteinaceous antigen to denaturing agents, whereas conformational epitopes are typically destroyed upon treatment with denaturing agents. An epitope comprises at least 3, at least 4, at least 5, at least 6, at least 7, or 8-10 amino acids in a unique spatial conformation.

The term "anti-drug antibody" as used herein denotes an antibody produced by the innate immune system of the recipient of a therapeutic antibody against said therapeutic antibody after administration thereof.

The term "immunogenicity" as used herein denotes the potential of a therapeutic antibody to induce an immune response in humans or animals. During drug development, immunogenicity is assessed primarily by the measurement of binding and neutralizing anti-drug antibodies.

A non-binding (germline) CDR denotes a CDR obtained from the human germline amino acid sequence to which the framework regions of the therapeutic antibody have the highest homology and which does not specifically bind to the target of the therapeutic antibody either alone or in combination with the other CDRs of the therapeutic antibody.

### METHODS ACCORDING TO THE INVENTION

1. A method for determining (the spatial location of) the epitope of an an anti-drug antibody, which is specifically binding to a therapeutic antibody specifically binding to a therapeutic target, comprising the following steps:
   a) separately incubating aliquots of a sample (that has been obtained from an (experimental) animal to which the therapeutic antibody had been administered), which comprises serum and the antibody specifically binding to a therapeutic antibody, separately with
      i) at least a Fab fragment of the therapeutic antibody, and
      ii) at least a Fab fragments of the therapeutic antibody in which all hypervariable regions (HVRs) have been replaced with non-binding (germline) HVRs, and
      and detecting the binding or non-binding of the anti-drug antibody specifically binding to a therapeutic antibody to the at least a Fab fragment in any of i) to ii),
      and
   b) determining (the spatial location of) the epitope of the anti-drug antibody specifically binding to a therapeutic antibody to be
      - in the HVRs that have been replaced in ii) if binding is detected in i)
         and
         non-binding is detected in ii).
2. The method according to item 1, wherein each HVR is a complete HVR determined according to the combined Kabat-Chothia definition.
3. The method according to any one of items 1 to 2, wherein the HVRs are determined according to the combined Kabat-Chothia definition and HVR-L1 encompasses residues 24-34, HVR-L2 encompasses residues 50-59, HVR-L3 encompasses residues 89-97, HVR-H1 encompasses residues 26-35, HVR-H2 encompasses residues 50-65 and HVR-H3 encompasses residues 95-102.

### EXEMPLARY EMBODIMENTS AND EXPERIMENTAL RESULTS

It is expressly pointed out that the following is presented simply as an exemplification of the method according to the current invention. It shall not be construed as a limitation. The scope of the invention is set forth in the claims.

The method according to the current invention will be exemplified in the following using a bispecific Fab, that has been designed and optimized to simultaneously recognize two individual targets, as example for a therapeutic antibody. A major intended application of these molecules is in the indication of Ophthalmology, like age-related macular degeneration (AMD) or diabetic macula edema (DME), where simultaneous suppression of two soluble factors (e.g. a neovascularization-promoting factor and an inflammation-mediating factor) is believed to have a beneficial/additive/synergistic/superior effect as compared to standard monotherapy alone. Bispecific Fabs show major advantages especially for Ophthalmology applications, as they combine two targeting sites within a comparably small molecular size (approx. 50 kDa), leading to a favorable drug:target ratio. Furthermore, they are highly concentratable fully engineerable towards desired specificity, affinity, stability and PK properties.

The method according to the invention was applied for the analysis of the immunogenicity response in a preclinical non-human primate study, involving 8 cynomolgus monkeys, which were dosed with a therapeutic bispecific Fab fragment (see example section for details). An ADA response was observed after intravitreal application of the molecule. With the method according to the invention, an in-depth characterization of the immunogenic response was possible and thereby predominant immunogenic parts of the therapeutic antibody were identified.

### Design of sequence variants

In general, not naturally occurring CDR and framework region sequences expected to be involved in binding to either antigen-1 or antigen-2 were individually analyzed and separately replaced by human germline sequence offering the closest sequence match. It has to be pointed out that for the replacement not each not-contiguous part of a paratope is necessarily replaced with stretches from the same, i.e. a single, human germline gene. More generally, the replacement of the different non-contiguous stretches is by sequence originating from different human germline sequences. Optionally, centrally located and generally buried residues in the variable domain can be retained in order to maintain the stable core packing of the VH-VL pair and optimally conserve the shape of the Fab fragment whilst exchanging the binding surface.

Generally, for achieving the back-to-germline modification of the drug antibody in question (germlinization) the variable domains are split up in different parts, as framework-regions as well as variable regions have to be germlined.

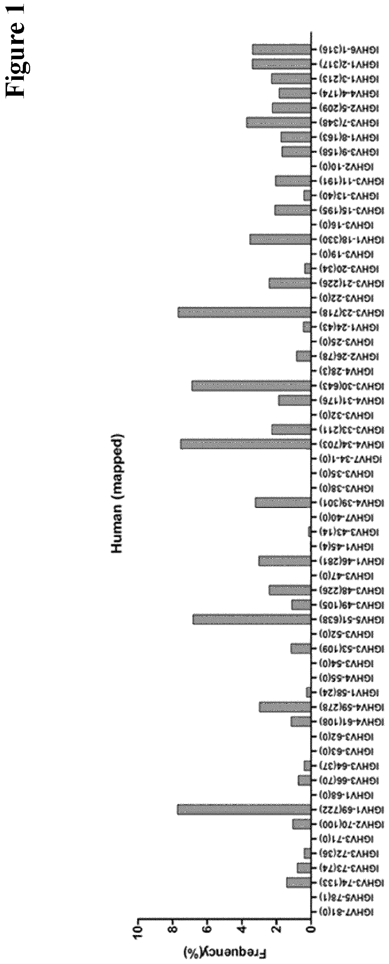

For the germlinization of frameworks either the N-terminal half of FR1 or the C-terminal half of FR3 of the drug antibody to be germlined is aligned against the human germline repertoire using a standard tool, such as e.g. Blast. The respective other part of FR1 and FR3 as well as FR2 are obtained by the germlinization of the respective CDRs. The sequence with the highest number of identical residues at identical positions is chosen as replacement sequence. If two or more like-identical germline sequences are identified the sequence with the higher usage frequency in the native human antibody repertoire is chosen. As basis for the usage frequency, the listing as provided in B. Shi, et al., can be used (Ther. Biol. Med. Model. 11 (2014) 30, supplemental Figure 1A; reproduced as Figure 1).

For the germlinization of HVRs the sequences corresponding to half the sequence of the N-terminally adjacent (preceding) and C-terminally adjacent (succeeding) framework, respectively, of the drug antibody are aligned together with a gap for the HVR against the human germline repertoire using a standard tool, such as e.g. Blast. The HVRs are determined according to the combined Kabat-Chothia definition, i.e. HVR-L1 are residues 24-34, HVR-L2 are residues 50-59, HVR-L3 are residues 89-97, HVR-H1 are residues 26-35, HVR-H2 are residues 50-65 and HVR-H3 are residues 95-102. The sequence with the highest number of identical residues outside the HVR at identical positions and having identical HVR length is chosen. If two or more germline sequences are identified the sequence with the higher usage frequency in the native human antibody repertoire is chosen. As basis for the usage frequency the listing as provided in B. Shi, et al. is used (Ther. Biol. Med. Model. 11 (2014) 30, supplemental Figure 1A; reproduced as Figure 1). Although the sequence of the respective HVRs is not used for the alignment to identify the respective most identical germline framework sequence, the respective HVR sequences in the identified germline sequences are used to replace the HVRs of the antibody to be germlined.

Heavy chain HVR-H3 is due to the VDJ-rearrangement that is taking place during antibody maturation formed by 1) a part of the V-element, 2) the D-element, and 3) part of the J-element. In more detail, the heavy chain HVR-H3 is generated by combining residues from a variable (VH) gene segment, a diversity (D) gene segment, and a joining (J) gene segment. Despite this already generated diversity by using different building blocks, additional diversity can be generated during the joining process with the addition of short palindromic nucleotides to the ends of the coding sequences or by deletion of a variable number of nucleotides from the ends of the coding segments or by subsequent insertion of a variable number of non-templated nucleotides at the VH-D and D-J junctions by terminal deoxynucleotidyl transferase or by the hypermutational machinery, which introduces point mutations to change amino acid codons (see Rosner, K., et al., Immunol. 103 (2001) 179-187).

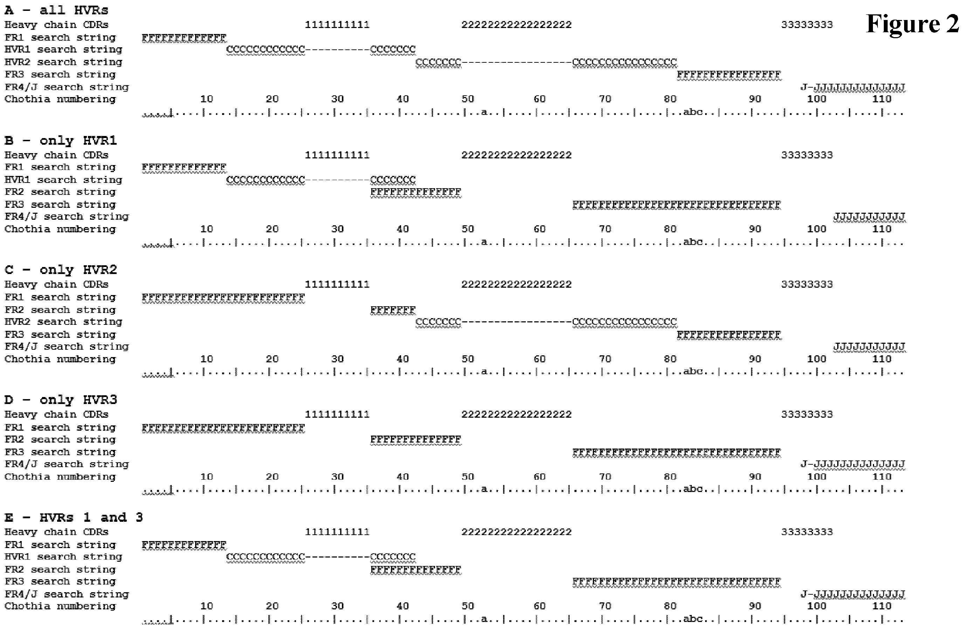

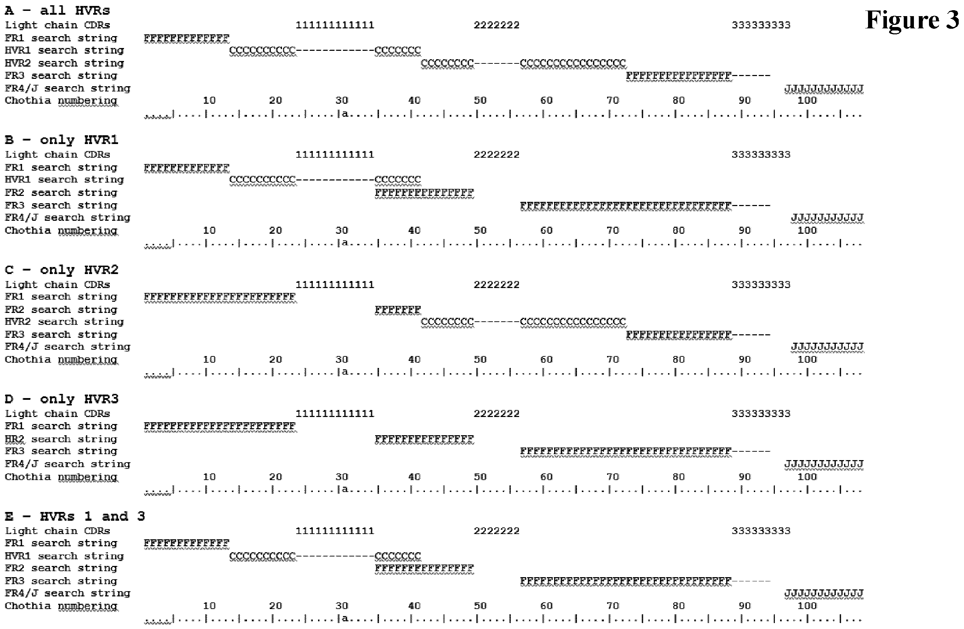

The general scheme for the germlinization is outlined in Figure 2A and 3A.

Thus, herein is provide as one advantage of the current method an easy-to-use method for paratope germlinization.

The exemplary bispecific Fab used in this example comprises a single pair of a VH domain and a VL domain that specifically binds to two different epitopes. One paratope comprises amino acid residues from HVR-H2, HVR-L1 and HVR-L3 and the other paratope comprises amino residues from HVR-H1, HVR-H3 and HVR-L2.

Thus, in this exemplary case only parts of the HVRs and FRs as shown in Figures 2C and 3E for the first paratope and in Figures 2E and 3C for the second paratope are germlined.

Thus, in order to create the Germ1-control construct, in which only the antigen-1 binding paratope is maintained and the antigen-2 binding paratope is removed, i.e. germlined, the following modifications have been made:
- HC-FR1 N-terminal part is replaced by germline sequence VH3-21 (IMGT),
- HC-FR1 C-terminal part, HVR1 and HC-FR2 N-terminal part are replaced by germline sequence VH3-23 (IMGT),
- HC-FR3 is replaced by germline sequence VH3-23 (IMGT),
- potentially immunogenic residue 96D has been replaced by a non-immunogenic A in accordance with used practices, such as in alanine-scans,
- HC-HVR3 residue 98-102 and FR4 are replaced by IGHJ4 (IMGT),
- LC-HVR2 is replaced by IGKV1-33 (IMGT).

Thus, in order to create the Germ2-control construct, in which only the antigen-2 binding paratope is maintained and the antigen-1 binding paratope is removed, i.e. germlined, the following modifications have been made:
- HC-FR2 C-terminal part, HVR2 and HC-FR3 N-terminal part are replaced by germline sequence VH3-23 (IMGT),
- LC-FR1 N-terminal part is replaced by germline sequence IGKV1-6 (IMGT),
- LC-FR1 C-terminal part, HVR1 and LC-FR2 N-terminal part are replaced by germline sequence VGKV1-27 (IMGT),
- LC-FR3 is replaced by germline sequence IGKV1d-43 (IMGT),
- LC-HVR3 residue 97 and FR4 are replaced by IGKJ2 (IMGT).

In order to create the GermGerm-control construct, all of the substitutions outlined above for the Germ1-control and Germ2-control constructs were combined, thus eliminating both the antigen-1 binding paratope and the antigen-2 binding paratope from the bispecific Fab.

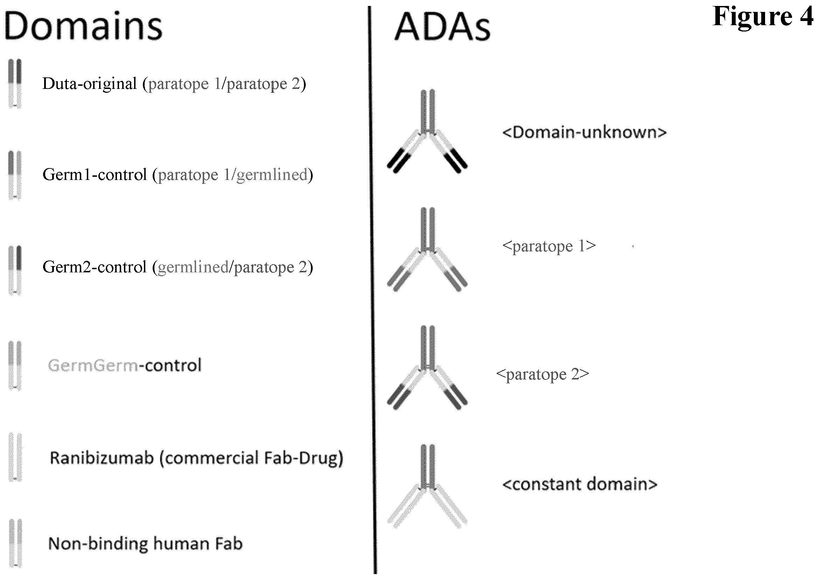

All constructs generated and applied for immunogenicity response characterization in this study are schematically illustrated in Figure 4.

The domain detection assay (DDA) according to the current invention employs the same principle as conventional bridging ADA assays. The inventive difference lies in the use of defined drug domains as capture reagents. Thereby it is possible to selectively determine domain-specific ADA fractions.

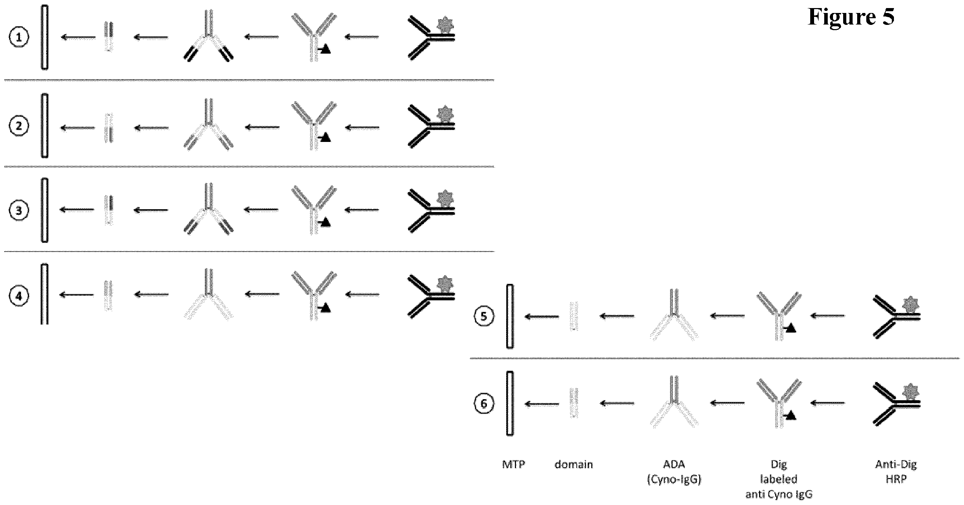

In one preferred embodiment of the method according to the current invention, the specific domains are directly immobilized to a solid surface (see Figure 5). Exemplary materials therefore are Nunc MaxiSorp^{™} MTPs. In one preferred embodiment, a digoxygenin-labeled anti-cynomolgus-IgG is used for detection of domain-specific ADA responses.

Besides the ADA domain detection assay according to the current invention, several other assay setups were applied for sample analysis and assessment of data reliability.

In a domain competition assay (DCA) setup, biotin and digoxygenin labeled variants of the different domains depicted in Figures 4 and 5 were used to form drug/drug variant:ADA complexes, which can be immobilized to a streptavidin-coated MTP and detected by an anti-Dig-HRP antibody. Non-labeled versions of the respective constructs were used as specific competition reagents. This assay yielded comparable results as compared to the domain detection assay according to the current invention, BUT is however much more laborious in terms of reagent generation and execution. Due to its indirect working mode (a signal reduction corresponds to the detection of domain-specific ADAs), it is also less sensitive. In terms of workload and quality of results, the DCA proved to be inferior to the DDA according to the current invention for ADA characterization.

A further assay applied for ADA characterization was an ADA bridging assay. In this assay, biotin- and digoxygenin-labeled versions of the drug candidate respective to the different control constructs were used to generate a signal, in case anti-drug antibodies are present in the sample. In order to confirm the specificity of the ADAs, a large surplus of unlabeled drug respective control construct is spiked. Significant signal quenching ability of the unlabeled molecules is considered as positivity for presence of the specific ADA. In the current example, this principle worked for the original bispecific Fab as well as for both partially germlined variants (one binding specificity active, the other binding-non-functional), but not for the GermGerm variant and the other control constructs, suggesting the absence of ADAs specific for the constant part. This finding is considered as a liability/weakness of the ADA bridging assay design, which may not be able to fully address the sparer and lower affine ADAs directed against the constant antibody portion (cf. Figure 4).

A third assay applied for ADA characterization of cynomolgus monkey study samples was an immune complex assay (see, e.g., Wessels, U., et al., Bioanal. 10 (2018) 803-814), which used a combination of an anti-human Fab antibody as capture and an anti-cynomolgus IgG antibody as detection reagent for detecting ADA:drug complexes in a biological sample. As this assay is not very drug tolerant, a positive signal was generated even with minute amounts of residual drug (Fab-original) still present in the sample, thus making it impossible to differentiate between the different constructs.

The domain detection assay according to the current invention, i.e. the combination of assay and the corresponding molecular engineering approach (generation of germlined variants of original drug compound), lacks all of the before outlined limitations, as the different domains are directly immobilized on the solid surface, such as, e.g., an MTP, and recognized by the respective ADAs. This makes the assay very specific and sensitive. The assay has also been shown to be highly drug tolerant, as concentration of the capture antibody is at least a factor of 200 higher compared to the residual drug levels present in the samples at day 35 post dose. This was also confirmed by titration experiments, showing that specific ADA recognition signals were still highly positive, when the drug was diluted out of the sample.

In terms of biological relevance and further development, the finding of a differentiated immune response to (predominantly) the antigen-binding parts and, with lower prevalence, the constant part of the bispecific Fab molecule supports the assumption of a complex polyclonal anti-drug antibody response against the fully human drug candidate in a non-human primate background. The finding, which ADAs directed against the constant part of the bispecific Fab molecule are also cross-reactive to the non-binding human Fab and Ranibizumab is especially notable with respect to the intended human application, as Ranibizumab is an intravitreally applied medication, which is well tolerated by humans. The results obtained therefore provide no hint for molecule-intrinsic immunogenic properties in the bispecific Fab modality.

### CONCLUSIONS AND OUTLOOK

Herein is reported a novel and inventive concept to facilitate the characterization of immunological responses against a biotherapeutic drug candidate in a preclinical setting. This is achieved by combining a molecular engineering approach with an efficient and easy to perform domain detection assay setup.

By replacing individual HVR and framework region sequences of the biotherapeutic drug candidate by human germline sequences, a novel and inventive assay tool has been generated that enabled the development of a highly specific and sensitive ADA domain characterization assay according to the current invention. This method according to the current invention can be used for assessment of the immunogenicity response from samples of a preclinical non-human animal, such as, e.g., primate, study.

The assay enabled the differentiation of anti-drug antibodies directed against all major domains of the biotherapeutic, i.e. the different constant parts or the paratopes, and also provided insights into the cross-reactivity behavior of anti-drug antibodies by using different control molecules, including a commercial Fab molecule approved for human treatment.

The approach described is applicable to all kinds of therapeutic antibodies, especially to smaller-sized protein biotherapeutics such as scFv or Fabs, as there is no need to enzymatically cleave the molecules into sub fragments prior to ADA characterization. In terms of data quality, robustness and ease-of-use, the assay proved to be superior compared to other bioanalytical methods.

From a projects perspective, early incorporation of germlined variants of clinical lead candidates as part of developability activities can add great value in terms of immunogenicity understanding and risk mitigation, as molecules bearing an intrinsic immunological risk can be identified early and excluded from further development in favor of more promising candidates.

The following examples, sequences and figures are provided to aid the understanding of the present invention, the true scope of which is set forth in the appended claims.

### Descrintion of the Figures

- **Figure 1**: Gene usage frequencies observed for the VH domain of human (n=9340) Ig sequences published in the IMGT/LIGM-DB database. Each of the mapped IGHV genes usage was calculated as the percentage of the total unique population of productive and in-frame sequences according to IMGT/HighV-QUEST Statistical Analysis Report (reproduced from B. Shi, et al., Ther. Biol. Med. Model. 11 (2014) 30, supplemental Figure 1A).
- **Figure 2**: Germlinization scheme heavy chain variable domain. The respective stretches of the heavy variable domain denoted with "F", "C" and "-" as well as "J" are used as query sequence in the alignment with the human IMGT germline repertoire to identify the most identical germline sequence. "-" represent a gap used in the alignment whereas the corresponding sequence of the identified germline is used to replace the respective HVR sequence of the antibody.
- **Figure 3**: Germlinization scheme light chain variable domain. The respective stretches of the light variable domain denoted with "F", "C" and "-" as well as "J" are used as query sequence in the alignment with the human IMGT germline repertoire to identify the most identical germline sequence. "-" represent a gap used in the alignment whereas the corresponding sequence of the identified germline is used to replace the respective HVR sequence of the antibody.
- **Figure 4**: Schematic illustration of the bispecific Fab biotherapeutic, engineered Fab variants and control molecules generated for detection of domain-specific anti-drug antibodies (ADAs). Constructs 2-4 are modified versions of original Duta-original drug molecule.
- **Figure 5:**: Domain detection assay principle: distinct domains (described in Figure 4) are directly coated to a Nunc MaxiSorp^{™} plate and used to capture domain-specific ADAs. Bound ADAs are detected by a Dig-labeled anti-Cynomolgus IgG. Signals are generated using an anti-Dig antibody coupled to horseradish peroxidase (HRP), leading to substrate/ABTS color conversion.
- **Figure 6:**: Schematic representation of an exemplary domain detection assays according to the current invention as used in example 5. Five different Fab variants, representing different domains are immobilized on the surface. ADAs with different specificities can attach to different domains. The further recognition of the ADAs is done by an anti-Cynomolgus antibody.
- **Figure 7:**: Expected positivity patterns of the exemplary assay setup of the method according to the current invention as used in example 5.
- **Figure 8:**: ADA-response "type 8" determined with the assay according to the invention.
- **Figure 9:**: ADA-response "type 9" determined with the assay according to the invention.
- **Figure 10:**: ADA-response "type 10" determined with the assay according to the invention.
- **Figure 11:**: ADA-response "type 11" determined with the assay according to the invention.

### EXAMPLES

### Chemicals, reagents and equipment

The therapeutic antibodies and specific assay reagents were all provided by Roche Diagnostics GmbH, Penzberg, Germany, and stored in aliquots at -80 °C until use. The bispecific therapeutic Fab ("Fab-original", drug) consists of a monoclonal Fab directed against two different antigens, which was available from recombinant expression. It was used after purification and analytical characterization. Biotinylated (Fab-original-Bi) and digoxygenylated (Fab-original-Dig) versions of the bispecific construct "Fab-original" were used as capture and detection reagents, respectively, in the bridging ADA assay.

For domain detection assay (DDA) and domain competition assay (DCA), the following un-labeled reagents were used for capturing: "Fab-original" (bispecific therapeutic Fab), "Germ1-control" (bispecific therapeutic Fab, wherein the first paratope is intact and the second paratope had been back-mutated to a germline sequence) and "Germ2-control" (bispecific therapeutic Fab, wherein the second paratope is intact and the first paratope was back-mutated to a germline sequence), "GermGerm-control" (bispecific therapeutic Fab, wherein both paratopes were back-mutated to a respective germline sequence), Ranibizumab (commercial human Fab biotherapeutic approved for intraocular application), a non-binding human Fab (DP47). A digoxygenylated monoclonal anti-cynomolgus IgG antibody was used as detection reagent. A polyclonal sheep antibody Fab against digoxygenin conjugated to horseradish peroxidase (HRP) (pAb-Dig-S-Fab-HRP) was used as a second detection reagent in all ADA assays.
Positive control, PC = generated in vivo or via phage-display
Capture control, CC = surrogate for PC when PC is not available

The following compounds served as positive controls (PC), e.g. as (functional (cloning, generation, immobilization) capture controls (CC): total ADA screening assay: monoclonal mouse antibody directed against human IgG kappa (mAb-anti-hu-kappa, Roche Diagnostics GmbH, Mannheim, Germany); DDA: digoxygenylated recombinant antigen-1 and digoxygenylated recombinant antigen-2 were used as capture controls (detection via anti-Ckappa), pre-dose cynomolgus monkey CTAD (CTAD = citrate-theophylline-adenine-dipyridamole; anticoagulant) plasma samples from each animal were used as negative control.

Pooled cynomolgus monkey CTAD plasma was prepared from 40 individual drug-naive female and male animals. Plasma samples were obtained from Sera Laboratory International, Ltd., Haywords Heath, UK. The washing buffer (phosphate-buffered saline (PBS)/0.05% Tween 20/0.002% Bronidox) and 2,2'-azino-bis-3-ethylbenzthiazoline-6-sulphonic acid (ABTS) substrate were provided by Roche Diagnostics GmbH, Mannheim, Germany. Ready to use LowCross Buffer was obtained from Candor Bioscience GmbH, Wangen, Germany, and was used as dilution and assay buffer in the bridging ADA assay (ELISA). All chemicals were of analytical grade.

Three dose groups of four animals (adult monkeys; cynomolgus monkeys) each were treated with 0 (placebo), 5 and 10 mg/eye for up to 43 days. A second treatment was administered at day 29. From this study, 39 plasma samples of bispecific Fab-original construct-dosed animals were used for ADA testing. Out of these, 31 samples were found positive in the total ADA screening assay (samples tested with a dilution factor 1:20). Finally, 16 samples from 8 different animals (from each animal the pre-dose sample as a specific negative control and day-35-after-treatment-sample) were selected for further evaluation by the domain-specific approaches because of significant signal intensities (>1.0 AU) reflecting high ADA concentrations.

Streptavidin-coated microtiter plates (SA-MTP) for the bridging ADA assay were obtained from Microcoat Biotechnologie GmbH, Bernried, Germany. Un-coated Nunc MaxiSorp^{™} microtiter plates for the DDA were obtained from Thermo Fisher Scientific, Germany.

### Example 1

### Bridging ADA assay for detection of the overall ADA response (ADA screening assay)

A bridging ADA assay was applied to analyze plasma samples obtained from a cynomolgus monkey study (see above) for detection of ADA responses directed against the bispecific <AG1/AG2> Fab (anti-antigen-1/antigen-2 bispecific Fab). The samples were tested at a dilution factor of 1:20. The bridging ADA assay served as a reference for the domain-specific approaches.

The bridging ADA assay is a sandwich enzyme linked immunosorbent assay (ELISA). Generally, antibody preparations were made in dilution buffer (LowCross Buffer, Candor Bioscience GmbH, Wangen, Germany). Reagents and samples were incubated at room temperature with shaking at 500 rpm. Washing steps consisted of three cycles of applying 300 µL of washing buffer followed by a final aspiration step. Generally, 15 µL of samples were added to 285 µL of incubation mixture containing 0.5 µg/mL each of Duta-original-Bi and Duta-original-Dig in a polypropylene preincubation plate. The PC (mAb-anti-hu-kappa M-1.7.10) was processed in parallel. After 1 h of incubation, 100 µL of each sample mixture were transferred in duplicate to a SA-MTP for 1 h of further incubation. After washing with PB S/Tween to remove unbound material and a final aspiration, 100 µL of the polyclonal anti-Dig-S-Fab-HRP conjugate (12.5 mU/mL) were added. After 1 h of incubation, the SA-MTP was washed three times followed by final aspiration. Then the substrate solution ABTS was added and the HRP catalyzed a color reaction with readout at 405 nm wavelength (reference wavelength: 490 nm). Only samples containing bridged complexes, i.e., antibodies bound to both the Duta-original-Bi and Duta-original-Dig, were able to generate signals. The signal intensities were proportional to the amount of ADAs present. Absorbance values were determined in duplicate wells for each sample. The absorbance values were averaged and accepted if the precision of the mean value was ≤20% CV. The screening cut point (CP) was evaluated according to Shankar et al., using the 95% percentile applied to 40 drug-naive donor samples. By calculating a plate specific CP (blank of pooled plasma multiplied by a normalization factor), negative samples (< plate-specific CP) and positive samples (> plate-specific CP) were identified. In this context, it should be mentioned that drug tolerance was not considered a crucial parameter. The drug is administered intravitreal, and study samples in this equation are taken at time points with low drug levels, i.e. prior to subsequent dosing. Since Fab-original levels in the samples were B.L.Q., an evaluation of drug tolerance was considered unnecessary.

### Example 2

### Generation of bispecific and variant Fab fragments

Synthetic genes encoding the bispecific construct Duta-original and the monospecific, partly germlined constructs Germ1-control and Germ2-control as well as the fully germlined, non-binding construct GermGerm-control were purchased from Geneart.

Synthetic antibody genes were cloned into a vector, which is a bicistronic vector for periplasmic expression of Fab fragments in E.coli, and bears an expression cassette comprising a LacZ promoter, LC ribosome binding site, LC signal peptide, Vk variable domain, Ck constant domain, HC ribosome binding site, HC signal peptide, VH variable domain, IgG1 CH1 constant domain and IgG1 upper hinge.

Plasmids encoding Fab fragments were transformed into TG1 E.coli cells (Zymo Research), and single colonies were pre-cultured at 37 °C in TB media, supplemented with 2% glucose to inhibit antibody expression. Upon reaching lag phase, pre-cultures were diluted in Erlenmeyer shaker flasks into TB expression media supplemented with a final concentration of 0.05% glucose, and upon reaching log phase expression cultures were induced by supplementation with IPTG to a final concentration of 1 mM. Fab fragments were expressed for 16 hours at 30 °C, and culture supernatants were clarified by centrifugation.

In order to ensure only full-length Fab fragments with intact heavy and light chain constant domains were used in the ADA analysis, the Fab fragments were double affinity purified in two steps, firstly using CaptureSelect IgG-CH1 resin and secondly using CaptureSelect Kappa XL resin, both purchased from GE Healthcare. The protein concentration of purified Fab fragments was determined by spectrophotometry, using absorbance at 280 nm.

### Example 3

### Domain detection assay (DDA) for detection of domain-specific ADA responses

The domain detection assays (DDAs) represented a four-step sandwich enzyme linked immunosorbent assay (ELISA).

Each 3.33 µg/mL and 150 µL per well of Fab-original, Germ1-control, Germ2-control, GermGerm-control, Ranibizumab and a non-binding human Fab were incubated for 1 hour on a Nunc MaxiSorp^{™} MTP as capturing reagents. After washing the plate 3 times with PBS/Tween to remove unbound material, 150 µL assay buffer was used to block the plate for 30 minutes to avoid unspecific binding from the samples in the next step. After washing the plate, 3 µL of each sample was diluted with 297 µL assay buffer and 100 µL per sample in duplicate were incubated for one hour. The capture controls (recombinant antigen-1-Dig and recombinant antigen-2-Dig) were processed in parallel. The pre-dose sample of each animal represented the negative control. The digoxygenylated monoclonal anti-cynomolgus IgG was used as detection reagent (100 µL/well). Wells with capture controls were filled in with 100 µL assay buffer. The polyclonal sheep antibody Fab fragment against Dig conjugated to horseradish peroxidase (HRP) (pAb-Dig-S-Fab-HRP) was applied as a second detection reagent in all ADA assays (100 µL/well). ABTS was added to each well and the ensuing color reaction was monitored by photometrical readout at 405 nm (reference wavelength 490 nm).

To differentiate between positive and negative samples, a cut-point (CP) determination was performed with the set of 8 pre-dose samples of the cynomolgus monkey study for each of the 5 DDAs.

**Table: Capture control assays used for the 5 different DDAs with expected positivity/negativity.**

| Controls | F(ab)<AG1/AG2> | Germ<AG1>-control | Germ<AG2>-control | GermGerm-control | Ranibizumab |
|---|---|---|---|---|---|
| Capture control 1 (AG1-Dig) | + | + | - | - | + |
| Capture control 2 (AG2-Dig) | + | - | + | - | - |
| Capture control 3 (anti-hu-kappa-Dig) | + | + | + | + | + |

All samples are measured in 3 dilutions (1 to 100 / 1 to 1000 / 1 to 10000). The samples were diluted in Low Cross Buffer^{™} and measured in parallel in all 5 assays.

### Example 4

### Capture control assays (CCA)

In order to test the reproducibility of the coating procedure for the 5 different DDAs, three different binding control assays were established. First, Nunc MaxiSorp^{™} plates were coated as described in the DDAs. Subsequently, digoxygenin labeled anti-human kappa light chain antibody or digoxygenin labeled antigen-1 or antigen-2 were incubated at a concentration of 50 ng/mL. A polyclonal sheep antibody Fab fragment against digoxygenin conjugated to HRP (pAb<Dig>S-Fab-HRP) was applied as a second detection reagent. ABTS was added to each well and the ensuing color reaction was monitored by photometrical readout at 405 nm (reference wavelength 490 nm). These assays were used to check the reproducibility of the coating and the functionality of the coated proteins. In addition, during the sample measurement, the capture control 1 assay was performed on the measuring plate as quasi-positive control ("surrogate PC") to ensure the comparability of individual measurements on different plates. The signal of this CCA was monitored and the assay was stopped at signal intensities of 1.8 - 2.2 OD₄₀₅ₙₘ.

### Example 5

### Characterization of the domain detection assay (DDA) according to the invention

The samples used were taken from a preclinical tolerability study in cynomolgus monkeys with the bispecific anti-antigen-1/antigen-2 Fab as a drug. Based on ADA positivity in the initial bridging assay for ADA screening (Wessels, U., et al., Bioanal. 10 (2018) 803-814), 16 plasma samples from 8 different animals treated with bispecific anti-antigen-1/antigen-2 Fab were selected for ADA characterization with the method according to the current invention. From each animal, the pre-dose sample as a specific negative control and the Day-35 post dose sample was tested. Pre dose cynomolgus monkey citrate-theophylline-adenosine-dipyridamole (CTAD) plasma samples from each animal were used as negative control for the DDA.

### Assay qualification

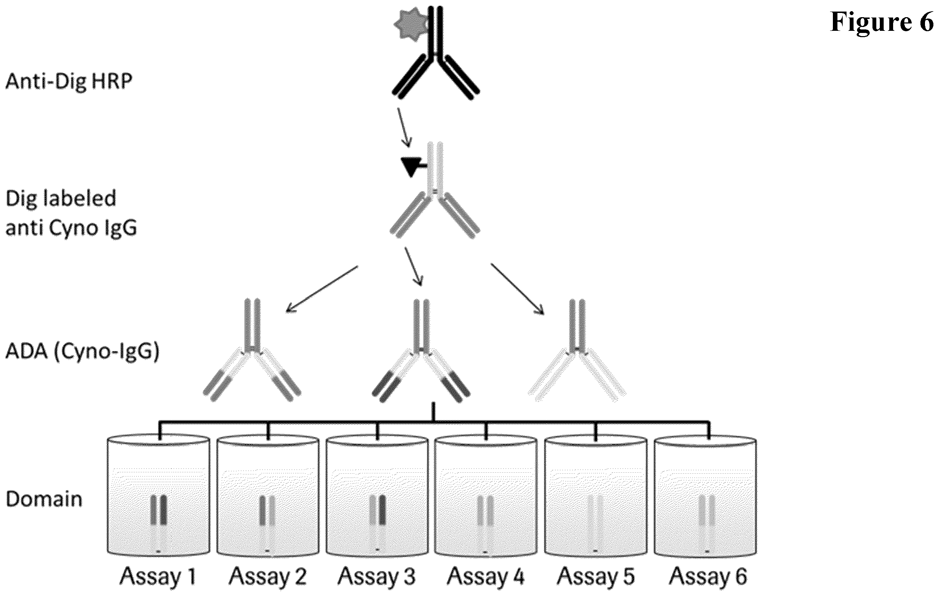

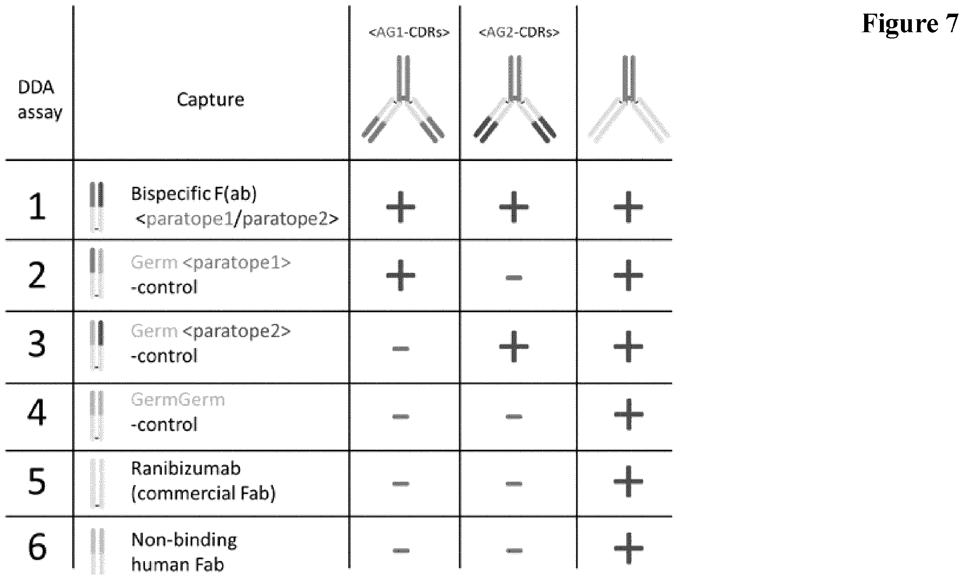

The example of the method according to the invention described in this example is for characterization of the ADA response and is based on the result of 5 individual DDAs being analyzed in relation to each other (see Figure 6 and Figure 7 showing the setup used in this example and the expected positivity pattern). This requires the generation of 5 distinct positive controls. This is a general challenge with DDAs and not limited to the assay described in this publication (Gorovits, B., et al., J. Immunol. Meth. 408 (2014) 1-12; Hock, M.B., et al., AAPS J. 17 (2015) 35-43). For an optimal qualification of these assays, specific positive controls would have to be generated, which can be achieved by immunization of mice with the complete compound and a good screening concept. Another possibility would be the use of already available antibodies against individual domains (Stubenrauch, K., et al., J. Pharm. Biomed. Anal. 114 (2015) 296-304). Due to the characteristics of the Fab therapeutic used in this example, the second approach was not possible, and unfortunately specific immunizations are very time consuming and laborious. Without positive controls, a standard approach for qualification of these assays was not possible. Therefore, a combination of capture control assays and an assay specific cut point determination was used as practical approach leading to reliable data.

### Capture consistency

The data from the capture control assay allows for the determination of a precision value. This precision value reflects the reproducibility of the coating. The anti-kappa IgG antibody based CCA is a measurement showing efficiency of Fab surface coating. This value was determined for all 5 individual DDAs. The AG1 and AG2 based CCAs also show whether the CDRs of the bound Fab fragments are accessible for either the antigen or potential ADAs. Precision data shows very little variation of 1 to 3 % for all 3 CCAs in 3 different intra assay runs.

**Table: Precision data for all 3 capture control assays in the five Domain detection assays.**

| **Control** (50 ng/ml) | F(ab)<AG1/AG2> | Germ<AG1>-control | Germ<AG2>-control | GermGerm-control | Ranibizumab |
|---|---|---|---|---|---|
| **Capture control AG1-Dig** | | | | | |
| Intra-assay precision (% CV) | 1% | 1% | -* | -* | 1% |
| Inter-assay precision (% CV) | 5% | 1096 | -* | -* | 4% |

| **Capture control AG2-Dig** | | | | | |
|---|---|---|---|---|---|
| Intra-assay precision (% CV) | 2% | -* | 296 | -* | -* |
| Inter-assay precision (% CV) | 3% | -* | 9% | -* | -* |

| **Capture control anti-human-kappa-Dig** | | | | | |
|---|---|---|---|---|---|
| Intra-assay precision (% CV) | 1% | 3% | 2% | 1% | 2% |
| Inter-assay precision (% CV) | 2% | 2% | 2% | 2% | 4% |

| | | | | | |
|---|---|---|---|---|---|
| * construct without binding competency for the respective target | | | | | |

It can be seen that the amount of coated antibody as well as their ability to bind the antigens is constant. This robustness in the coating is a prerequisite for the comparability of the data. This data also shows that the used constructs all have or do no longer have the desired functionalities.

### DDA cut point determination

A study specific cut point was calculated for all 5 DDAs to assess the ADA positivity by measuring the corresponding pre-dose samples (n=8 animals). For the calculation of the cut point, the 99 % percentile was used. Multiplicative normalization factors as well as corresponding cut points for all assays are listed in the following Table.

| Parameter | F(ab)<AG1/AG2> | Germ<AG1>-control | Germ<AG2>-control | Germ Germ-control | Ranibizumab |
|---|---|---|---|---|---|
| MRD | 100 | 100 | 100 | 100 | 100 |
| Sample volume for double determination | | | 3 µL for each assay | | |
| Normalization factor | 1.48 | 1.40 | 2.00 | 1.66 | 1.34 |
| Assay signals at screening cut point | 0.117 | 0.206 | 0.159 | 0.104 | 0.049 |

The assay range was determined by the use of digoxygenin-labelled antigens (antigen-1 and antigen-2). At a concentration of 50 ng/ml antigen-1-Dig or antigen-2-Dig the assay was developed until a signal of 1.8-2.2 OD₄₀₅ₙₘ was reached. This signal range was used as a criterion to stop the reaction in the real sample measurement. Real samples were diluted 1:100 in assay buffer. In case signals above 2.2 OD₄₀₅ₙₘ were reached, samples were further diluted in 1:10 steps.

A cut-point determination was performed with the respective pre-dose samples for each individual construct.

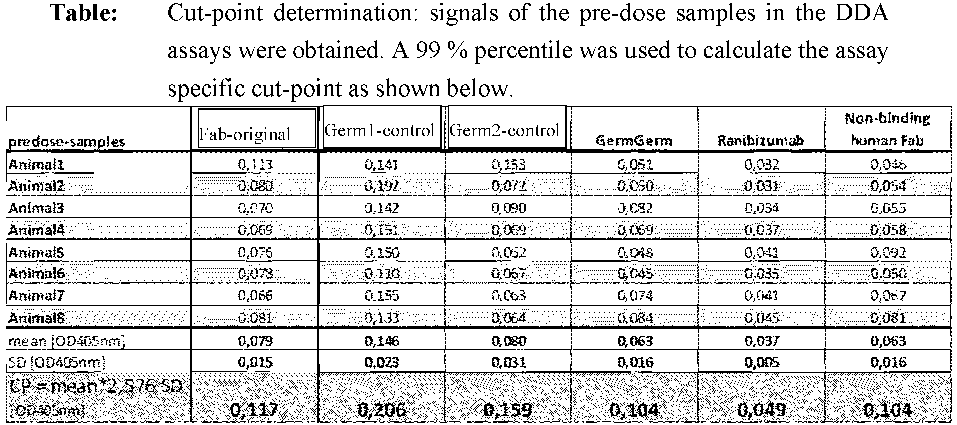

### Drug tolerance of DDAs

The samples that were used are from late time points (day 35 after drug administration). Therefore, the measured levels of residual drug are very low and no impact of residual drug on these assays is to be expected. To discuss this theoretically, it has to be said that in this assay the labelled drug is coated with a very high concentration, which is in direct competition with the residual drug in the samples. The theoretical drug tolerance should therefore be at least as good as with a standard bridging assay. If necessary, this can be improved in a similar way to bridging ADA assays. For example, the samples can be pre-treated with acid to dissolve existing drug-ADA complexes (see, e.g., Kavita, U., et al., J. Immunol. Meth. 448 (2017) 91-104).

The assay has been shown to be highly drug tolerant, as mass concentration of the capture antibody/domain was orders of magnitude higher as compared to the residual drug levels present in the samples at day 35 post dose. This was also confirmed by titration experiments, showing that specific ADA recognition signals were still highly positive, when the drug was diluted out of the sample.

### Sample analysis with Domain Detection Assays

The numerical data for all samples with all 3 dilutions used can be found in the following table.

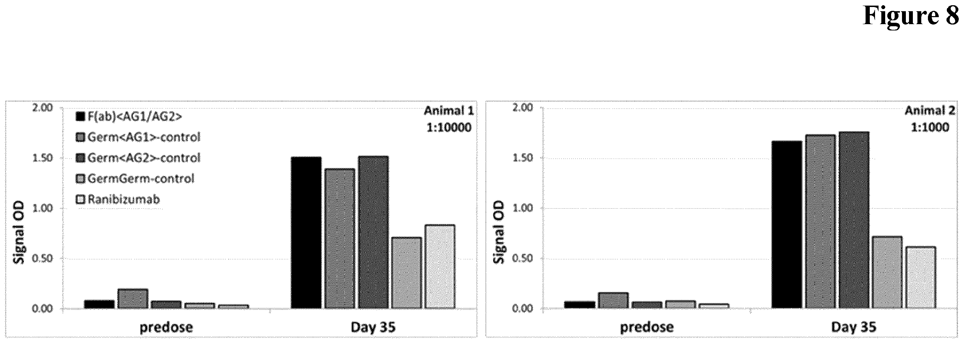

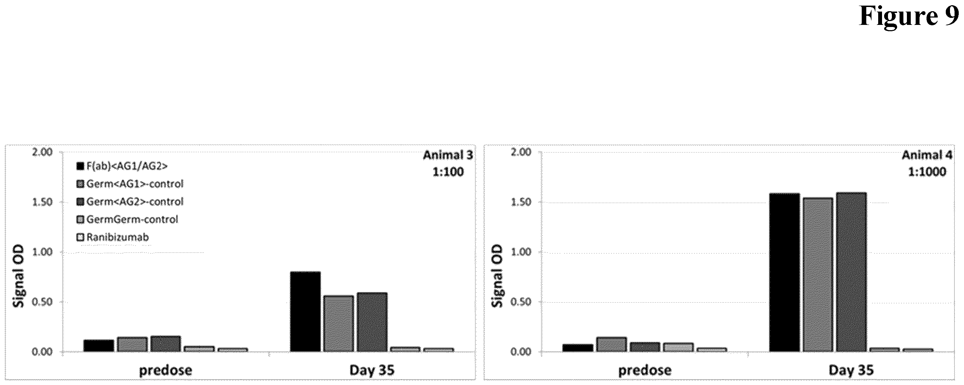

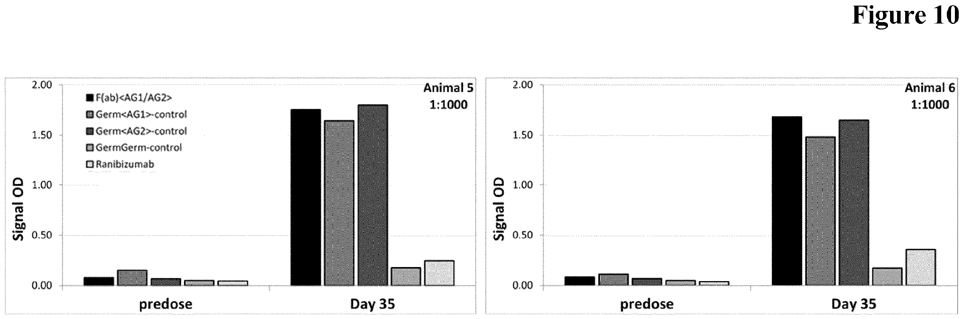

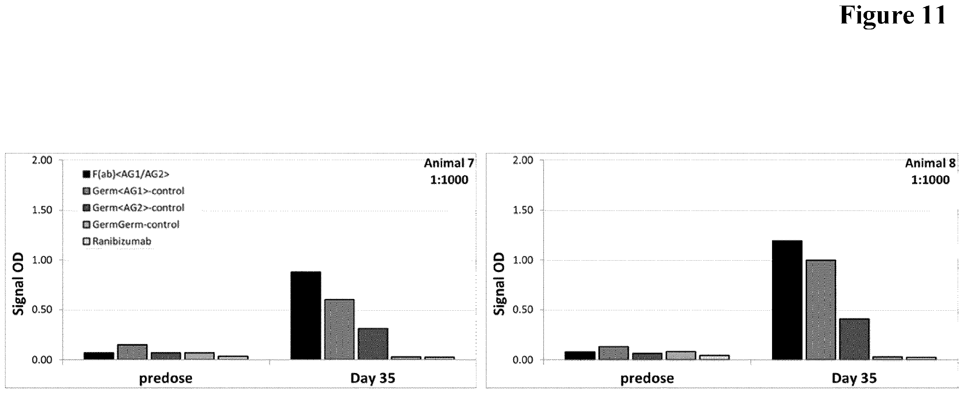

**Table: Numeric ADA domain detection assay results. Values used for graphical illustration in Figures 8 to 11 are depicted in bold.**

| | mean signals OD (405nm) | | | | | | | |
|---|---|---|---|---|---|---|---|---|
| | Animal 1 | | | | Animal 2 | | | |
| ADA response against | predose (1100) | postdose (1:100) | postdose (1:1000) | postdose (1:10000) | predose (1:100) | postdose (1:100) | postdose (1:1000) | postdose (1:10000) |
| Bispecific F(ab) <AG1/AG2> | **0.080** | 1.756 | 1.704 | **1.505** | **0.066** | 1.738 | **1.663** | 1.418 |
| Germ<AG1>-control | **0.192** | 1.824 | 1.810 | **1.389** | **0.155** | 1797 | **1.725** | 1,259 |
| Germ<AG2>-control | **0.072** | 1.886 | 1.850 | **1.513** | **0.063** | 1.860 | **1.753** | 1.163 |
| GermGerm-control | **0.050** | 1.906 | 1.740 | **0.707** | **0.074** | 1.666 | **0.714** | 0.112 |
| Ranibizumab (commercial Fat | **0.031** | 1.951 | 1.888 | **0.831** | **0.041** | 1674 | **0.611** | 0.082 |

| | | | | | | | | |
|---|---|---|---|---|---|---|---|---|
| mean signals OD [405nm] | | | | | | | | |

| | Animal 3 | | | | Animal 4 | | | |
|---|---|---|---|---|---|---|---|---|
| ADA response against | predose (1:100) | postdose (1:100) | postdose (1:1000) | postdose (1:10000) | predose (1:100) | postdose (1:100) | postdose (1:1000) | postdose (1:10000) |
| Bispecific F(ab) <AG1/AG2> | **0.113** | **0.797** | 0.171 | 0.035 | **0.070** | 1,687 | **1.581** | 0.992 |
| Germ<AG1>-control | **0.141** | **0.557** | 0.078 | 0.029 | **0.142** | 1.690 | **1.537** | 0.563 |
| Germ<AG2>-control | **0.153** | **0.588** | 0.089 | 0.032 | **0.090** | 1.770 | **1.590** | 0.645 |
| GermGerm-control | **0.051** | **0.041** | 0.030 | 0.028 | **0.082** | 0.098 | **0.033** | 0.026 |
| Ranibizumab (commercial Fat | **0.032** | **0.032** | 0.022 | 0.022 | **0.034** | 0.160 | **0.026** | 0019 |

| | | | | | | | | |
|---|---|---|---|---|---|---|---|---|
| mean signals OD [405nm] | | | | | | | | |

| | Animal 5 | | | | Animal 6 | | | |
|---|---|---|---|---|---|---|---|---|
| ADA response against | predose (1:100) | postdose (1:100) | postdose (1:1000) | postdose (1:10000) | predose (1:100) | postdose (1:100) | postdose (1:1000) | postdose (1:10000) |
| Bispecific F(ab) <AG1/AG2> | **0.076** | 1.769 | **1.751** | 1.246 | **0.078** | 1.758 | **1.679** | 0.905 |
| Germ<AG1>control | **0.150** | 1.755 | **1.642** | 0.856 | **0.110** | 1.742 | **1.478** | 0.468 |
| Germ<AG2>-control | **0.062** | 2.009 | **1.799** | 0.788 | **0.067** | 2.011 | **1.647** | 0.510 |
| GermGerm-control | **0.048** | 1.215 | **0.175** | 0.039 | **0.045** | 1296 | **0.170** | 0.039 |
| Ranibizumab (commercial Fat | **0.041** | 1.312 | **0.244** | 0.037 | **0.035** | 1.509 | **0.354** | 0.045 |

| | | | | | | | | |
|---|---|---|---|---|---|---|---|---|
| mean signals OD [405nm] | | | | | | | | |

| | Animal 7 | | | | Animal 8 | | | |
|---|---|---|---|---|---|---|---|---|
| ADA response against | predose (1:100) | postdose (1100) | postdose (1:1000) | postdose (1:10000) | predose (1:100) | postdose (1:100) | postdose (1:1000) | postdose (1:10000) |
| Bispecific F(ab) <AG1/AG2> | **0.069** | 1.571 | **0.879** | 0.150 | **0.081** | 1776 | **1.192** | 0.269 |
| Germ<AG1>-control | **0.151** | 1.548 | **0.601** | 0.097 | **0.133** | 1.649 | **0.996** | 0185 |
| Germ<AG2>-control | **0.069** | 1.405 | **0.314** | 0.053 | **0.061** | 1460 | **0.104** | 0.066 |
| GermGerm-control | **0.069** | 0.058 | **0.031** | 0.029 | **0.084** | 0.083 | **0.031** | 0.027 |
| Ranibizumab (commercial Fab | **0.037** | 0.084 | **0.026** | 0.023 | **0.045** | 0100 | **0.025** | 0.019 |

Different dilutions were selected for the comparison of the data with each other as shown in Figures 8 to 11. The linear range of the chromogenic ABTS substrate is limited and the samples must be diluted to different extents to make the signal heights clearly distinguishable. For 6 of 8 animals, the 1 to 1000 dilution was used. In animal 3, the immune response was weak and the 1 to 100 dilution was chosen and in animal 1 the 1 to 10000 dilution was chosen because the immune response was very strong.

Four ADA response patterns (in the following termed 8, 9, 10 and 11 in line with the corresponding results in figures 8 to 11) can be discriminated which are representative for all eight ADA positive animals of the study.

Pattern 8: Animals 1 and 2 reveal a mixed ADA response against different parts (CDRs and backbone) of the molecule. A strong response against the fully binding competent <AG1/AG2> bispecific F(ab), the Germ<AG1>-control and the Germ<AG2>-control can be seen. A moderate response was detected against the fully germlined control construct (GermGerm-control) and Ranibizumab, indicating the presence of ADAs against the constant regions of the molecule. The majority of ADAs appears to be directed against both CDRs in similar proportions.

Pattern 9: Animals 3 and 4 show only a recognition of the CDR regions with no ADAs against the constant regions. In animal 3, the signal against both CDRs here is lower than in Pattern A, suggesting a more moderate ADA response, whereas animal 4 reveals a similar signal strength as Pattern A. In these two animal also, a similar number of ADAs against both CDRs appear to exist.

Pattern 10: Animals 5 and 6 are similar to pattern 8, indicating a mixed response against both CDRs and the constant regions. However, a much lower proportion of ADAs against the constant regions of the molecule was detected as compared to pattern 8.

Pattern 11: Animals 7 and 8 show no ADAs against the constant regions and only against the CDRs. Unlike Pattern 9 and all other animals, we see a different distribution of ADAs between the two CDRs against antigen 1 and 2. There seem to be more ADAs against the AG1 binding site.

Overall, all pre-dose samples were ADA negative in all five DDAs. Therefore, we conclude that all observed immunogenicity responses from the Day-35 post-dose cynomolgus study samples are treatment-related ADA responses.

The signals of the pre-dose samples were also very well comparable with small variations in all 5 DDAs. Thus, these values were used to calculate DDA specific in study cut points (see above: DDA cut point determination). For this proceeding, the number of baseline samples used is relevant and the recommendation (Shankar, G., et al., J. Pharm. Biomed. Anal. 48 (2008) 1267-1281; Amaravadi, L., et al., Bioanal. 7 (2015) 3107-3124) is to use at least 50 pre-dose samples. This is not possible with significantly lower animal numbers in many preclinical studies such as this. Nevertheless, given the exploratory nature of this measurement, this approach has been chosen.

Summarizing the above, with the method according to the current invention all eight animals were found to show ADAs against the CDRS and only in two of eight animals, these responses were slightly different. Due to this mixed immune response, it is not likely that one of the two CDR regions of the bispecific molecule was predominantly responsible for the observed immune responses. It was not observed that individual domains exhibit strongly different immunogenicity.

One more point that had to be addressed was the question whether the engineered bispecific F(ab) fragment used here, with its slightly different structure to a wild type Fab fragment, bears a higher immunogenicity risk. Four out of eight animals showed antibodies against the constant regions. In no sample higher signals in the DDA with the Germ-Germ variant were found compared to the DDA with Ranibizumab. This would be evidence of an immune response against a neoepitope only present on the Germ-Germ variant which does not exist on Ranibizumab, an ocular therapeutic known for its low immunogenicity, as shown in several clinical studies (Figurska, M., et al., Klin. Oczna. 112 (2010) 147-150).

Based on the above findings, all immunogenicity responses from the above cynomolgus study are treatment-related ADA responses. The dual/bispecific Fab specificity of the treatment-induced ADAs was confirmed. Overall, the immunogenicity response is directed against the HVRs and the constant Fab part. In 50 % of the animals, a purely "monospecific" response against the HVRs is observed, whereas in the other half a mixed response against both HVRs and backbone is seen.

No ADA response against the constant backbone without HVR contribution is observed. Notably, anti-drug antibodies directed against the constant part of the bispecific Fab are cross-reactive to classical human Fab molecules like the non-binding (DP47) control molecule and Ranibizumab. This is an important result towards a potential human application, as there is no hint for molecule-intrinsic immunogenic properties in the bispecific Fab.

## Claims

1. A method for determining the epitope of an anti-drug antibody, which is specifically binding to a therapeutic antibody specifically binding to a therapeutic target, comprising the following steps:
a) separately incubating aliquots of a sample, which comprises the anti-drug antibody, with
i) at least a Fab fragment of the therapeutic antibody, and
ii) at least a Fab fragment of the therapeutic antibody in which all hypervariable regions (HVRs) of the paratope to the therapeutic target have been replaced with germline or non-binding HVRs,
and detecting the binding or non-binding of the anti-drug antibody to the at least a Fab fragment in any of i) to ii),
and
b) determining the epitope of the anti-drug antibody to be
- in the paratope to the therapeutic target if binding is detected in i)
and
non-binding is detected in ii).

2. The method according to claim 1, wherein the non-binding or germline HVR is obtained from the same germline as the Framework-regions of the therapeutic antibody are.

3. The method according to any one of claims 1 to 2, wherein the therapeutic antibody is a mono- or bispecific Fab.

4. The method according to any one of claims 1 to 3, wherein the paratope is formed by a VH/VL-pair.

5. The method according to any one of claims 1 to 4, wherein
- the therapeutic antibody is a bispecific Fab, wherein the first paratope is formed by the HVRs H-1, H-3 and L-2 and the second paratope is formed by the HVRs H-2, L-1 and L-3,
- in step a) step ii) is
the therapeutic antibody in which HVRs H-1, H-3 and L-2 have been replaced with non-binding HVRs,
- step a) further comprises
vi) the therapeutic antibody in which HVRs H-2, L-1 and L-3 have been replaced with non-binding HVRs,
- step b) is
determining the epitope of the anti-drug antibody to be
- in the first paratope of the bispecific Fab if binding is detected in i) and vi),
and
non-binding is detected in ii),
- in the second paratope of the bispecific Fab if binding is detected in i) and ii),
and
non-binding is detected in vi),
- in the HVR-Framework region-junction of the first paratope if binding is detected in i) and vi)
and
non-binding is detected in ii),
- in the HVR-Framework region-junction of the second paratope if binding is detected in i) and ii)
and
non-binding is detected in vi).

## Patentansprüche

1. Verfahren zum Bestimmen des Epitops eines Anti-Drug-Antikörpers, der spezifisch an einen therapeutischen Antikörper bindet, der spezifisch an ein therapeutisches Ziel bindet, umfassend die folgenden Schritte:
a) separates Inkubieren aliquoter Teile einer Probe, die den Anti-Drug-Antikörper umfasst, mit
i) mindestens einem Fab-Fragment des therapeutischen Antikörpers, und
ii) mindestens einem Fab-Fragment des therapeutischen Antikörpers, in dem alle hypervariablen Regionen (HVRs) des Paratops an das therapeutische Ziel durch Keimbahn- oder nichtbindende HVRs ersetzt worden sind,
und Nachweisen des Bindens oder Nichtbindens des Anti-Drug-Antikörpers an das mindestens eine Fab-Fragment in einem von i) bis ii),
und
b) Bestimmen, dass sich das Epitop des Anti-Drug-Antikörpers
- im Paratop an das therapeutische Ziel befindet, wenn in i) Binden nachgewiesen wird und in ii) Nichtbinden nachgewiesen wird.

2. Verfahren nach Anspruch 1, wobei die nichtbindende oder die Keimbahn-HVR aus derselben Keimbahn wie die Gerüstregionen des therapeutischen Antikörpers erhalten wird.

3. Verfahren nach einem der Ansprüche 1 bis 2, wobei der therapeutische Antikörper ein mono- oder bispezifisches Fab ist.

4. Verfahren nach einem der Ansprüche 1 bis 3, wobei das Paratop von einem VH/VL-Paar gebildet wird.

5. Verfahren nach einem der Ansprüche 1 bis 4, wobei
- der therapeutische Antikörper ein bispezifisches Fab ist, wobei das erste Paratop von den HVRs H-1, H-3 und L-2 gebildet wird und das zweite Paratop von den HVRs H-2, L-1 und L-3 gebildet wird,
- in Schritt a) Schritt ii)
der therapeutische Antikörper der ist, in dem HVRs H-1, H-3 und L-2 durch nichtbindende HVRs ersetzt worden sind,
- Schritt a) ferner Folgendes umfasst
vi) der therapeutische Antikörper ist der, in dem die HVRs H-2, L-1 und L-3 durch nichtbindende HVRs ersetzt worden sind,
- Schritt b) Folgendes ist
Bestimmen, dass das Epitop des Anti-Drug-Antikörpers
- in dem ersten Paratop des bispezifischen Fab vorliegt, wenn in i) und vi) Binden nachgewiesen wird,
und
in ii) Nichtbinden nachgewiesen wird,
- in dem zweiten Paratop des bispezifischen Fab vorliegt, wenn in i) und ii) Binden nachgewiesen wird,
und
in vi) Nichtbinden nachgewiesen wird,
- in dem HVR-Gerüstregion-Gelenk des ersten Paratops vorliegt, wenn in i) und vi) Binden nachgewiesen wird
und
in ii) Nichtbinden nachgewiesen wird,
- in dem HVR-Gerüstregion-Gelenk des zweiten Paratops vorliegt, wenn in i) und ii) Binden nachgewiesen wird
und
in vi) Nichtbinden nachgewiesen wird.

## Revendications

1. Procédé de détermination de l'épitope d'un anticorps anti-médicament, qui se lie spécifiquement à un anticorps thérapeutique se liant spécifiquement à une cible thérapeutique, comprenant les étapes suivantes :
a) incubation de manière séparée d'aliquotes d'un échantillon, qui comprend l'anticorps anti-médicament, avec
i) au moins un fragment Fab de l'anticorps thérapeutique, et
ii) au moins un fragment Fab de l'anticorps thérapeutique dans lequel toutes les régions hypervariables (HVR) du paratope de la cible thérapeutique ont été remplacées par des HVR de lignée germinale ou non liantes,
et détection de la liaison ou de l'absence de liaison de l'anticorps anti-médicament à l'au moins un fragment Fab de l'un quelconque de i) à ii),
et
b) détermination de l'épitope de l'anticorps anti-médicament comme étant
- dans le paratope de la cible thérapeutique si une liaison est détectée en i) et une absence de liaison est détectée en ii).

2. Procédé selon la revendication 1, dans lequel la HVR non liante ou de lignée germinale est obtenue à partir de la même lignée germinale que les régions cadres de l'anticorps thérapeutique.

3. Procédé selon l'une quelconque des revendications 1 à 2, dans lequel l'anticorps thérapeutique est un Fab mono- ou bispécifique.

4. Procédé selon l'une quelconque des revendications 1 à 3, dans lequel le paratope est formé par une paire VH/VL.

5. Procédé selon l'une quelconque des revendications 1 à 4, dans lequel
- l'anticorps thérapeutique est un Fab bispécifique, dans lequel le premier paratope est formé par les HVR H-1, H-3 et L-2 et le second paratope est formé par les HVR H-2, L-1 et L-3,
- dans l'étape a) l'étape ii) est
l'anticorps thérapeutique dans lequel les HVR H-1, H-3 et L-2 ont été remplacées par des HVR non liantes,
- l'étape a) comprend en outre
vi) l'anticorps thérapeutique dans lequel les HVR H-2, L-1 et L-3 ont été remplacées par des HVR non liantes,
- l'étape b) est
détermination de l'épitope de l'anticorps anti-médicament comme étant
- dans le premier paratope du Fab bispécifique si une liaison est détectée en i) et vi),
et
une absence de liaison est détectée en ii),
- dans le second paratope du Fab bispécifique si une liaison est détectée en i) et ii),
et
une absence de liaison est détectée en vi),
- dans la jonction région cadre-HVR du premier paratope si une liaison est détectée en i) et vi)
et
une absence de liaison est détectée en ii),
- dans la jonction région cadre-HVR du second paratope si une liaison est détectée en i) et ii)
et
une absence de liaison est détectée en vi).
